# EUROPÄISCHE PATENTSCHRIFT

(11) **EP 1 671 936 B1**
(45) Veröffentlichungstag und Bekanntmachung des Hinweises auf die Patenterteilung: **31.10.2007**
(21) Anmeldenummer: 05110587.2
(22) Anmeldetag: 10.11.2005
(51) Int. Cl.: C07C 29/04, C07C 29/09, C07C 29/10, C07D 301/16, C07C 31/18, A61K 8/34

(54) **Verfahren zur Herstellung von Alkandiolen und Alkantriolen mit einer vicinalen Diol-Gruppe**
Process for synthetizing paraffinic diols and triols possessing a vicinal diol group
Procédé de préparation d'alcanediols et d'alkanetriols à group diol vicinal

(30) Priorität: 15.12.2004 DE 102004060541
(43) Veröffentlichungstag der Anmeldung: 21.06.2006
(73) Patentinhaber: Degussa GmbH, 40474 Düsseldorf (DE)
(72) Erfinder: Osterholt, Clemens, 46286 Dorsten (DE); Brehme, Volker, 48155 Münster (DE); Kübelbäck, Thomas, 48249 Dülmen (DE); Köhler, Günther, 45770 Marl (DE)

(56) Entgegenhaltungen:
- WO-A-01/89466
- DE-A1- 1 944 120
- US-B1- 6 281 394
- PATENT ABSTRACTS OF JAPAN Bd. 2000, Nr. 19, 5. Juni 2001 (2001-06-05) & JP 2001 048720 A (ASAHI DENKA KOGYO KK), 20. Februar 2001 (2001-02-20)
- PATENT ABSTRACTS OF JAPAN Bd. 1999, Nr. 12, 29. Oktober 1999 (1999-10-29) & JP 11 193225 A (SHISEIDO CO LTD), 21. Juli 1999 (1999-07-21)
- DATABASE WPI Section Ch, Week 200415 Derwent Publications Ltd., London, GB; Class A96, AN 2004-147909 XP002387516 & JP 2003 342146 A (POLA CHEM IND INC) 3. Dezember 2003 (2003-12-03)

## Beschreibung

Die vorliegende Erfindung beschreibt ein Verfahren zur Herstellung von Diolen und Triolen durch Umsetzung von Alkenen bzw. Alkenolen mit Ameisensäure und wässriger Wasserstoffperoxidlösung. Die dabei als Zwischenstufe gebildeten Formiate werden durch anschließende Umesterung in die entsprechenden Diole und Triole überführt.

Aus der Literatur ist bekannt, 1,2-Diole durch Umsetzung von 1-Olefinen mit Ameisensäure und Wasserstoffperoxid bei einer Temperatur von 40 °C und anschließender Verseifung herzustellen (D. Swern et al. in J. Am. Chem. Soc. 68 (1946), Seiten 1504 bis 1507).

DE 19 44 120 beschreibt ein Verfahren zur Herstellung von Glycerin aus Allylacetat und wässriger Peressigsäure. Nach der destillativen Abtrennung der Leichtsieder in Form von nicht umgesetztem Allylacetat, Wasser und Essigsäure wird der verbleibende Glycerinessigsäureester (überwiegend Monoacetin) mit p-Toluolsulfonsäure versetzt und mit Methanol umgeestert. Nach der Umesterung wird der Katalysator mittels eines basischen lonentauschers aus dem Reaktionsgemisch entfernt und das Rohprodukt destillativ aufgearbeitet (Beispiele 1-7).
Nachteilig dabei ist der Einsatz des giftigen Methanols als Mittel zur Umesterung sowie der zusätzliche Aufarbeitungsschritt durch die Behandlung mit einem basischen lonentauscher, der insbesondere bei einem technischen Verfahren zu erheblichem Mehraufwand führt. Des weiteren verläuft die Oxidationsreaktion mit Essigsäure und Wasserstoffperoxid langsamer als mit Ameisensäure und Wasserstoffperoxid.

US 2,739,173 beschreibt ein Verfahren zur Herstellung von Glycerin aus Allylalkohol und wässriger Peressigsäure, bei dem zunächst eine Reaktionsmischung aus Glycerinformiaten erzeugt wird, aus der anschließend wässrige Ameisensäure abdestilliert wird und nach Zugabe von Methanol in den Destillationssumpf und Schwefelsäure durch Erhitzen Glycerin und Ameisensäuremethylester erzeugt und der entstandene Ester abdestilliert werden. Anschließend wird die Schwefelsäure mit NaOH neutralisiert, dann überschüssiges Methanol abdestilliert und das Glycerin aus dem Rückstand destillativ isoliert (Beispiel).
Nachteilig dabei ist wiederum der Einsatz des giftigen Methanols als Mittel zur Umesterung sowie der zusätzliche Aufarbeitungsschritt durch Neutralisation mit NaOH und der damit einhergehende Salzanfall. Insbesondere von Nachteil ist jedoch, dass die als Katalysator verwendete Schwefelsäure bei der Destillation die Riechqualität des Zielproduktes negativ beeinträchtigt vor allem bei Einsatz von Alkenolen mit mehr als 3 C-Atomen anstelle von Allylalkohol.

DE 32 29 084 beschreibt ein Verfahren zur Herstellung von vicinalen Diolen und deren Formiaten. Die bei der Umsetzung von Olefinen mit Ameisensäure und Wasserstoffperoxid gebildeten Formiate werden durch Decarbonylierung mit katalytischen Mengen Alkoholat in die entsprechenden Diole überführt.
Nachteilig dabei sind zum einen der Einsatz von Alkoholaten, die üblicherweise in Lösemitteln gehandhabt werden, wie beispielsweise Kalium-tert. Butylat, und deren Handhabung im technischen Verfahren aufwändig ist und zum anderen die hohen Reaktortemperaturen von 150 bis 180°C bei der Decarbonylierung und dadurch bedingte Beeinträchtigung der geruchlichen Qualität der Zielprodukte sowie das Entstehen von giftigem Kohlenmonoxid.

DE 29 37 840 beschreibt ein Verfahren zur Hydroxylierung von aliphatischen Mono- und Diolefinen. Es wird unter anderem die Umsetzung von unsubstituierten oder mit ein bis zwei Hydroxygruppen substituierten Monoolefinen mit Ameisensäure und Wasserstoffperoxid genannt. Nach der Umsetzung wird zum H₂O₂-Abbau das Rohprodukt üblicherweise über ein Festbett (Platin-Festbett-Kontakt) geleitet. Die als Zwischenstufe gebildeten Formiate (im Patent nicht speziell genannt) werden mit Wasser bzw. NaOH verseift.
Nachteilig dabei sind zum einen zum H₂O₂-Abbau zusätzlich benötigte Aufarbeitungsstufe an einem speziellen Festbettkatalysator (alle Beispiele). Zum anderen wird in den Beispielen zur Herstellung von Diolen mit ≥ 5 C-Atomen (Beispiele 3 - 6) mit NaOH verseift und das Produkt mit einem Lösungsmittel extrahiert, was einen zusätzlichen Aufwand und Verbrauch von Hilfsstoffen erfordert, sowie zwangsläufig zu Salzanfall führt.

EP 0 141 775 beschreibt ein kontinuierliches Verfahren zur Herstellung von 1,2-Alkandiolen. Dazu werden 1,2-Olefine mit Ameisensäure und Wasserstoffperoxid umgesetzt und die gebildeten Formiate in einem mehrstufigen Verfahren mit konzentrierten wässrigen Alkalilösungen verseift. Das Reaktionsprodukt wird anschließend mit einem organischen Lösemittel extrahiert und aus dem Extrakt durch Abdestillieren des Lösungsmittels isoliert.
Nachteilig dabei ist der notwendige Einsatz von Lösemitteln sowie das Entstehen von stöchiometrischen Mengen an Salzen der Ameisensäure, die abgetrennt und aufwändig entsorgt werden müssen.

GB 2 145 076 beschreibt ebenfalls ein kontinuierliches Verfahren zur Herstellung von 1,2-Alkandiolen. Dazu werden 1,2-Olefine mit Wasserstoffperoxid und Ameisensäure umgesetzt und die erhaltenen Ester mit 25 %iger Natriumhydroxidlösung verseift. Anschließend wird die organische Phase destilliert.
Nachteilig dabei ist wiederum das Entstehen der Ameisensäuresalze, die abgetrennt und aufwändig entsorgt werden müssen. Außerdem begünstigen hier Restmengen an Natriumformiat in der organischen Phase die Zersetzung der Diole bzw. Triole bei der anschließenden destillativen Aufarbeitung, so dass ein verunreinigtes Produkt entstehen kann, das insbesondere auch Geruchskomponenten enthalten kann.

DE 197 43 015 beschreibt ein Verfahren zur Herstellung von vicinalen Diolen oder Polyolen. Der durch Oxidation mit Wasserstoffperoxid/Ameisensäure primär gebildete Formylester der Diole oder Polyolen z.B. des Dodecen-1,2-diols, fällt in Form einer organischen und einer wässrigen Phase an. Gemäß Beispiel 1 enthält die wässrige Phase überschüssige Ameisensäure, die organische Phase immer noch 14 % Wasser und als organische Komponenten Formylester und 1,2-Diole. Im nächsten Schritt wird entweder nach Abtrennung der wässrigen Phase mittels Phasentrennung nur die wasserhaltige organische Phase oder ohne Abtrennung der wässrigen Phase das komplette Reaktionsprodukt - wie in Beispiel 3 - nach Zusatz von Schwefelsäure und Methanol thermisch behandelt. Dabei werden die Formylester der Diole oder Polyole hydrolysiert bzw. methanolysiert und anschließend bei Normaldruck Ameisensäuremethylester, Methanol und Wasser gemeinsam als Azeotrop abdestilliert.

Die vicinale Diole oder Polyole bleiben als Rückstand zurück.

### Weitere Nachteile dieses Verfahrens sind:

Die Umesterung mit Methanol erfolgt in jedem Fall in Gegenwart von Wasser und überschüssiger Ameisensäure, so dass durch die gleichzeitig stattfindende Veresterung der noch vorhandenen freien Ameisensäure zusätzlich Ameisensäuremethylester gebildet wird und gleichzeitig der Methanolverbrauch erhöht wird.
Außerdem fällt bei der Destillation ein Dreistoffgemisch aus Methanol, Wasser und Ameisensäuremethylester an, das zum Zweck der Recyclierung aufwändig getrennt werden muss.
Durch die hohe Temperatur in der Oxidationsreaktion von ca. 100°C zersetzt sich vermehrt Wasserstoffperoxid, so dass auch hier ein relativ hoher Verbrauch resultiert. Ein Absenken der Temperatur würde dort jedoch zu höheren Restperoxidgehalten führen, die bei der folgenden Hydrolyse/Umesterung bedingt durch die gleichzeitig anwesenden Niedrigsieder Methanol bzw. Ameisensäuremethylester zu erhöhten sicherheitstechnischen Risiken führen würden, so dass sich dieser Weg verbietet.
Die Umesterung und Hydrolyse mit Methanol in Gegenwart von Ameisensäure und Wasser unter Zusatz von Schwefelsäure führt zu einem Zielprodukt mit unangenehmem Geruch (nicht erfindungsgemäßes Beispiel 8), ähnlich wie auch die reine Umesterung mit Methanol unter Zusatz von Schwefelsäure (nicht erfindungsgemäßes Beispiel 3), so dass nach solchen Verfahren hergestellte Diole oder Triole für viele Anwendungsbereiche (z. B. in der Kosmetik) völlig ungeeignet sind.
Auch wird als Alkohol ausschließlich Methanol verwendet, der aufgrund seiner Giftigkeit in der Produktion nur ungern verwendet wird. Insbesondere bei der Verwendung der Diole oder Triole als Vorprodukte für die Kosmetikindustrie dürfen keine Methanolrückstände im Produkt verbleiben, was entsprechenden Reinigungsaufwand erforderlich macht. Zur Verwendung anderer Alkohole für die Umesterung wird in DE 197 43 015 jedoch keine Aussage gemacht.
Die Reaktion ganz ohne stark sauren Katalysator ist durch die langen Reaktionszeiten oder die bei entsprechender Temperaturerhöhung auftretenden typischen Nachteile wie vermehrte Bildung von Nebenprodukten bzw. Geruchskomponenten auch nachteilig. So sind gemäß Beispiel 3 und 7 zur Hydrolyse ausschließlich mit Wasser einmal große Mengen Wasser und sehr lange Reaktionszeiten von 17 bis 18 Stunden erforderlich.

DE 199 29 196 beschreibt ein Verfahren zur Herstellung von wasser-, säure- und geruchsfreien vicinalen Diolen, bei dem nach Oxidation des Olefins mit Wasserstoffperoxid/Ameisensäure oder Essigsäure und nach Zufügen eines Lösungsmittels Wasser und Ameisensäure als ternäres Azeotrop abdestilliert werden. Das zurückbleibende, die Formylester der Diole enthaltende Gemisch wird bezogen auf die Estergruppen mit ungefähr stöchiometrischen Mengen wässriger Natronlauge oder Natriumcarbonatlösung alkalisch verseift und anschließend nach Zugabe eines weiteren Schleppmittels, wie z.B. Toluol, das Wasser azeotrop abdestilliert. Das dabei ausgefallenen Natriumformiat wird abfiltriert (Beispiel 2). In einer anderen Variante wird die Toluol enthaltende Verseifungslösung mit HCl neutralisiert und das dann gebildete Salz abgetrennt (Beispiel 4). Die zurückbleibende Toluolphase wird eingeengt und das Diol isoliert.
Trotz der Angabe im Titel, dass das Verfahren zu geruchsfreien Diolprodukten führen würde, sind die Endprodukte aller angegebenen Beispiele nicht geruchsfrei, sondern zeigen beispielsweise fruchtigen oder fettig-aldehydigen Geruch (Beispiel 2). Die Anmelderin konnte in eigenen Versuchen bestätigen, dass bei alkalischer Verseifung der intermediären Diolameisensäureester Produkte mit unerwünschten Geruchsnoten erhalten werden. Außerdem ist das Verfahren der DE 199 29 196 sehr aufwändig und führt wie andere Verfahren dieser Gattung zu unerwünschten Salzfrachten.

Alle die nach dem Stand der Technik bekannten Verfahren haben entweder den Nachteil, dass sie technisch sehr aufwändig sind, oder zu Produkten mit nicht ausreichender Reinheit, insbesondere mit nachteiligen Geruchseigenschaften führen.

Es bestand daher die Aufgabe, ein möglichst einfaches im halbtechnischen und technischen Maßstab praktikables Verfahren zur Herstellung von vicinalen Alkandiolen oder Alkantriolen bereitzustellen, das insbesondere in gängigen technischen Anlagen durchführbar sein sollte, möglichst kurze Reaktionszeiten und hohe Raum-Zeit-Ausbeuten gewähren sowie die sicherheitstechnischen Anforderungen in einfacher Weise erfüllen sollte. Darüber hinaus sollte das Verfahren minimale Verluste an Hilfsstoffen wie Alkohol oder Säure, eine einfache Aufarbeitung ohne zusätzliche Lösungsmittel und einfache Destillation der Zielprodukte gewährleisten, sowie möglichst hohe Reinheiten, insbesondere hinsichtlich der geruchlichen Eigenschaften der gebildeten Diole und Triole. Es sollten insbesondere geruchsfreie vicinale Alkandiole oder Alkantriole auf möglichst einfache Weise hergestellt werden und die Nachteile der herkömmlichen Verfahren vermieden werden.

Gelöst werden diese sowie weitere nicht explizit genannten Aufgaben, die jedoch aus den hierin diskutierten Zusammenhängen ohne weiteres ableitbar oder erschließbar sind durch ein Verfahren nach Anspruch 1. Zweckmäßige Ausformungen und Abwandlungen des erfindungsgemäßen Verfahrens werden in den auf Anspruch 1 rückbezogenen Unteransprüchen unter Schutz gestellt.
Dadurch dass man, ein Verfahren zur Herstellung von vicinalen Alkandiolen oder Alkantriolen umfassend die Schritte
a1) Umsetzung entsprechender Monoolefine mit 4 bis 20 Kohlenstoffatomen mit Wasserstoffperoxid und Ameisensäure in Gegenwart von Wasser oder
a2) Umsetzung entsprechender Monoolefinalkohole mit 4 bis 20 Kohlenstoffatomen in einer ersten Stufe mit Ameisensäure und des in der ersten Stufe gebildeten Reaktionsproduktes in einer zweiten Stufe mit Wasserstoffperoxid in Gegenwart von Wasser,
b) Abdestillieren von Wasser und Ameisensäure aus der in Schritt a1) oder a2) erhaltenen Mischung,
c) Umsetzung der in Schritt b) erhaltenen Mischung mit mindestens einem aliphatischen C₁-C₄-Alkohol in Gegenwart von mindestens einer Arylsulfonsäure der Formel RₙAr-SO₃H, bei der R gleich oder verschieden sein können und einen linearen oder verzweigten C₁- bis C₁₉-Alkylrest, Fluor oder Chlor bedeuten, Ar ein Phenyl-, Naphthyl-, Anthryl- oder Phenanthrylrest ist und n = 0, 1, 2 oder 3 sein kann, oder von mindestens einer C₆- bis C₁₆-Alkylsulfonsäure und
d) Abdestillieren von in Schritt c) gebildetem Ameisensäureester zusammen mit ggf. noch vorhandenem Alkohol aus der Reaktionsmischung von Schritt c),
bei dem die Schritte c) und d) nacheinander oder gleichzeitig durchgeführt werden, anwendet, gelingt es, ein hochreines Produkt, welches keinerlei nachteilige Geruchseigenschaften aufweist, in sehr guten Ausbeuten zur Verfügung zu stellen und die Nachteile der bekannten Herstellungsverfahren zu überwinden.
Das erfindungsgemäß erzielte Ergebnis ist völlig überraschend, da aus DE 197 43 015 zwar die Verwendung von starken Säuren wie Schwefelsäure oder Flußsäure als Katalysator in einer Methanolysereaktion von Alkandiolformiaten zu Alkandiolen zur Beschleunigung der Reaktion bekannt war (Spalte 5, Zeile 60 bis 64), aber nirgends ein Hinweis darauf zu finden war, wie die nachteiligen Geruchseigenschaften der Produkte, welche durch Umesterung mit Schwefelsäure erzeugt werden, verhindert werden können. Die nachteiligen Geruchseigenschaften von schwefelsauer katalysiert hergestellten Produkten waren in der bisherigen Literatur gar nicht erwähnt worden.
Die in DE 197 43 015 erwähnte Möglichkeit der Verwendung von Flußsäure kommt schon deshalb nicht in Betracht, weil Flußsäure aufgrund ihrer Flüchtigkeit und ihrer toxischen und hautgefährdenden Eigenschaften ein höheres Risiko in der Produktion birgt und so ein überproportional großer zusätzlicher Sicherheitsaufwand im Betrieb notwendig wäre. Andere starke anorganische Säuren wie HCl oder organische Säuren wie Essigsäure führen zu einer zu niedrigen Reaktionsgeschwindigkeit, so dass überproportional hohe Temperaturen angewendet werden müssten, was die Nebenprodukt- und Geruchsbildung wiederum verstärken würde.
Das völlige Weglassen von Säure führt grundsätzlich zu deutlich längeren Reaktionszeiten von z.B. 17 bis 18 Stunden wie in der Hydrolysereaktion in Beispiel 3 bzw. 7 in der DE 197 43 015, was unerwünscht ist. Außerdem müssen in diesem Fall auch entsprechend höhere Temperaturen angewendet werden mit den bereits genannten nachteiligen Folgen.
p-Toluolsulfonsäure ist zwar grundsätzlich als saurer Katalysator für Veresterungsreaktionen bekannt, die erfindungsgemäße Wirkung der Erzielung geruchsfreier Produkte durch ein Verfahren nach dem Anspruch 1, bei dem die genannten Arylsulfonsäuren oder höheren Alkylsulfonsäuren als Umesterungskatalysator verwendet werden, konnte jedoch in keiner Weise erwartet werden.

Durch die gemeinsame destillative Abtrennung des vorhandenen sowie des in der Reaktion entstandenen Wassers und der Ameisensäure gemäß Schritt b) gelingt es, den Verbrauch an Alkohol in der nachfolgenden Umesterung gemäß Schritt c) deutlich zu senken. Ebenso wird der Zwangsanfall an Ameisensäurealkylester und damit der Verfahrensaufwand in Schritt c) reduziert. Auch entfallen die aufwändige Trennung eines Dreistoffgemisches aus Wasser, Methanol und Ameisensäureester, die gemäß DE 197 43 015 zunächst als Azeotrop abdestilliert werden, und die chemische Rückspaltung des zusätzlich gebildeten Ameisensäuremethylesters zu Methanol und Ameisensäure. Die erfindungsgemäß anfallende wässrige Ameisensäure kann hingegen ggf. nach erfolgter Aufkonzentration direkt in die Oxidationsreaktion zurückgeführt werden.
Gleichzeitig wird während der Destillation des Wasser/Ameisensäuregemisches bereits noch vorhandenes Wasserstoffperoxid weitgehend bis völlig thermisch zerstört, was einen hohen Gewinn für die Sicherheit darstellt. Die sonst so gefürchtete Bildung von Hydroperoxiden, die insbesondere während der Destillation eines Dreistoffgemisches aus Wasser, Alkohol und Ameisensäurealkylester bei Verwendung von im Vergleich zu Methanol höheren Alkoholen eine Rolle spielen kann, wird so zuverlässig verhindert.
Es kann jedoch zusätzlich von Vorteil sein, wenn die in Schritt b) erhaltene Mischung vor Schritt c) nochmals zur Zerstörung ggf. noch vorhandenen Wasserstoffperoxids oder organischer Hydroperoxide thermisch behandelt wird. Dies geschieht vorzugsweise bei Temperaturen von 70 bis 100°C, besonders bevorzugt bei ca. 80 °C und Verweilzeiten von ca. einer Stunde bei dieser Temperatur.

Erfindungsgemäß können sowohl lineare als auch verzweigte Monoolefine bzw. Monoolefinalkohole ggf. auch in Form von Isomerengemischen eingesetzt werden, die noch weitere unter Reaktionsbedingungen inerte Substituenten tragen können. Isomerengemische werden z.B. dann eingesetzt, wenn die resultierenden Mischungen aus isomeren Diolen bzw. Triolen auch ohne Isomerentrennung direkt weiterverwendet werden können.

Bevorzugt eingesetzt werden in Schritt a1) 2,4,4-Trimethyl-1-penten, 2,4,4-Trimethyl-2-penten oder Gemische aus beiden Isomeren, 1-Penten, 1-Hexen, 1-Octen oder 1-Dodecen und in Schritt a2) 3-Buten-1-ol, 5-Hexen-1-ol oder 9-Decen-1-ol.

Erfindungsgemäß bevorzugt eingesetzt werden in Schritt a2) mindestens 2 Molequivalente Ameisensäure, besonders bevorzugt jedoch 4 bis 10 Molequivalente Ameisensäure. Dadurch wird sichergestellt, dass die freie Hydroxygruppe größtenteils mit Ameisensäure verestert wird, wobei anteilig Wasser frei wird. Die Veresterung schützt dabei vor unerwünschten Oxidationsreaktionen bei der nachfolgenden Umsetzung mit Wasserstoffperoxid. Auf diese Weise wird eine größtmögliche Ausbeute und Reinheit der Produkte in den nachfolgenden Reaktionsschritten erreicht.

Dabei entsteht schon während der Zumischung der Ameisensäure zum Alkenol das entsprechende Alkenylformiat und der dabei erzielte Umsatz ist stark vom Mol-Verhältnis Ameisensäure : Alkenol abhängig.

Im Falle von 5-Hexen-1-ol wurden je nach Mol-Verhältnis von Ameisensäure : 5-Hexen-1-ol (= V) die folgenden Umsätze erreicht:
V = 1 : 1 Umsatz 52 %
V = 2 : 1 Umsatz 86 %
V = 4 : 1 Umsatz 96 %
V = 10:1 Umsatz > 99 %

Der Umsatz von Alkenol zu Alkenylformiat hat einen wichtigen Einfluss auf die Nebenkomponentenbildung bei der nachfolgenden Oxidationsstufe und somit auch auf die Gesamtausbeute. Je höher der Alkenolumsatz, desto weniger Nebenkomponenten werden dort gebildet und desto mehr kann die Gesamtausbeute gesteigert werden.
Erfindungsgemäß bevorzugt wird die Umsetzung mit Wasserstoffperoxid und Ameisensäure (Oxidation der Doppelbindung) in Schritt a1) oder in der zweite Stufe von Schritt a2) bei Temperaturen von 40 bis 120 °C, besonders bevorzugt von 50 bis 80 °C durchgeführt.

Nach erfolgter H₂O₂-Dosierung kann die Reaktion in einer Nachreaktionszeit von 0,5 bis 10 Stunden, vorzugsweise aber von 1 bis 2 Stunden bei der zuvor eingestellten Reaktionstemperatur vervollständigt werden.

Das dabei eingesetzte Wasserstoffperoxid wird sowohl in Schritt a1) als auch in Schritt a2) vorzugsweise in Form einer 30 - 70 %igen wässrigen Lösung, besonders bevorzugt in Form einer 50 - 70 %igen wässrigen Lösung eingesetzt.
Um eine Peroxid-Depot-Bildung bei diskontinuierlicher Reaktionsführung zu vermeiden, kann die H₂O₂-Dosierung in 4 bis 10 Stunden, bevorzugt innerhalb von 5 bis 7 Stunden erfolgen. Der Peroxidgehalt im Reaktor wird während der Dosierzeit vorzugsweise bei < 10 % gehalten. Die für die Reaktion insgesamt benötigte Wasserstoffperoxidmenge ist abhängig von der Beschaffenheit des Reaktors. Bei einem Reaktor mit glatter Oberfläche (z.B. Glas) ist ein Molverhältnis von Alken bzw. Alkenol : H₂O₂ von 1 : 1,2 ausreichend. Bei Reaktoren mit rauher Oberfläche ist, aufgrund von teilweiser H₂O₂-Zersetzung, ein größerer Überschuss erforderlich.
Die Reaktion wird im halbtechnischen und technischen Maßstab vorzugsweise in einem Reaktor mit einer glatten Oberfläche beispielsweise aus Emaille durchgeführt. Es ist zusätzlich vorteilhaft, den Reaktor vor der Oxidationsreaktion ausreichend zu passivieren. Dies kann zum Beispiel durch dem Fachmann bekannte Methoden, wie die Behandlung mit Phosphorsäure oder Durchführen einer ersten Oxidationsreaktion, deren Produkte nicht weiterverwertet werden, erreicht werden. Auf diese Weise können die Wasserstoffperoxidverluste durch Zersetzung in den folgenden Ansätzen weiter verringert werden.

Das Abdestillieren von Wasser und Ameisensäure in Schritt b) wird vorzugsweise bei einem Druck von 100 mbar bis 1 bar und einer Sumpftemperatur von 20 bis 150°C durchgeführt, besonders bevorzugt jedoch bei einem Druck von 200 mbar bis 800mbar und einer Sumpftemperatur von 60 bis 120°C.

Das Gemisch kann durch dem Fachmann bekannte Maßnahmen weiter aufkonzentriert werden und insbesondere die Ameisensäure in die Oxidationsreaktion zurückgeführt werden. Dabei ist kein Ameisensäureester anwesend, was eine deutliche Vereinfachung der Aufarbeitung beispielsweise im Vergleich zu DE 197 43 015 bewirkt.

Als aliphatischen C₁-C₄-Alkohol in Schritt c) wird dabei bevorzugt Methanol, Ethanol, n-Propanol, iso-Propanol, n-Butanol, sek.-Butanol oder tert.-Butanol eingesetzt. Alle genannten Alkohole sind preiswert und in verschiedenen Reinheitsgraden erhältlich, die je nach geforderter Produktspezifikation eingesetzt werden können. Außerdem gelingt erfindungsgemäß mit diesen Alkoholen die Alkoholysereaktion in schneller und effizienter Weise.

Besonders bevorzugt eingesetzt werden jedoch Ethanol, n-Propanol und/oder iso-Propanol, die eine vergleichsweise geringe Toxizität aufweisen. Ganz besonders bevorzugt wird Ethanol eingesetzt, bei dem auch ein möglicher Verbleib von Spuren im Endprodukt relativ unkritisch ist und der Reinigungsaufwand entsprechend geringer ausfallen kann.

Als Arylsulfonsäure in Schritt c) werden erfindungsgemäß vorzugsweise Arylsulfonsäuren der Formel RₙAr-SO₃H, bei denen Ar Phenyl- oder Naphthylrest ist, bevorzugt Benzolsulfonsäure, p-Toluolsulfonsäure und/oder Marlon-AS3-Säure eingesetzt.
Als Alkylsulfonsäure in Schritt c) werden erfindungsgemäß bevorzugt Hexansulfonsäure und/oder Dodecansulfonsäure eingesetzt.
Ganz besonders bevorzugt wird Marlon-AS3-Säure (CAS-Nr.: 85536-14-7) eingesetzt, die eine in 4-Position mit sekundären C₁₀₋₁₃-Alkylresten substituierte Benzolsulfonsäure der folgenden Formel darstellt: 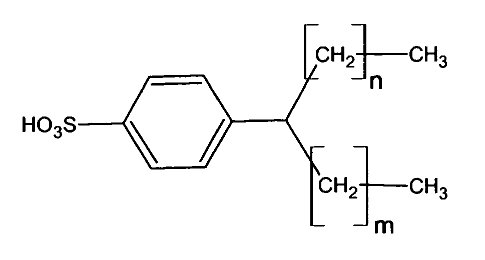 wobei m + n = 7 bis 10 bedeuten.

Durch Verwendung der genannten Säuren gelingt neben einer schnellen und effizienten Umesterungsreaktion auch die Bereitstellung von Endprodukten die nahezu bis völlig geruchlos sind. Dies ist neu und völlig überraschend und stellt einen außerordentlichen Vorteil dar, weil die Verwendung der erfindungsgemäß hergestellten vicinalen Diole bzw. Triole in der Kosmetikindustrie so überhaupt erst ermöglicht wird.

Der Katalysator in Schritt c) wird dabei vorzugsweise in einer Menge von 0,005 bis 1 Mol.-%, bevorzugt von 0,01 bis 0,30 Mol.-% bezogen auf die Menge an eingesetztem Monoolefin bzw. Monoolefinalkohol verwendet. Die optimale Konzentration kann der Fachmann je nach den weiteren Randbedingungen leicht ermitteln.

Die Umsetzung mit C₁-C₄-Alkohol (Umesterung) in Schritt c) und die Abtrennung des Ameisensäureesters erfolgt vorzugsweise bei einer Sumpftemperatur von 40 bis 160 °C, besonders bevorzugt bei einer Sumpftemperatur von 50 bis 110 °C, jedoch ganz besonders bevorzugt bei einer Sumpftemperatur von 60 bis 90 °C. Selbst bei Temperaturen im oberen Vorzugsbereich werden beim erfindungsgemäßen Verfahren Produkte ohne nachteilige Geruchseigenschaften erhalten. Möglichst niedrige Temperaturen bei der Umsetzung und/oder der Destillation des gebildeten Ameisensäurealkylesters sind dabei vorteilhaft, weil dabei die Tendenz zur Geruchsbildung noch weiter reduziert werden kann.

Bei diskontinuierlichen Ausführung der Umesterung werden vorteilhaft die in Schritt b) erhaltenen Formiatzwischenprodukte und der Katalysator vorgelegt, die Mischung je nach verwendetem Alkohol auf die gewünschte Reaktionstemperatur eingestellt und der Alkohol in den unteren Teil des Reaktors eingespeist. Das dabei entstehende Alkylformiat wird zusammen mit überschüssigem Alkohol über Kopf einer Destillationsvorrichtung abgezogen. Der Umsatzverlauf wird über geeignete Messmethoden verfolgt (z.B. über Gaschromatographie oder Verseifungszahl) und die Umsetzung bei einem > 90 %igen, besonders bevorzugt jedoch bei einem > 99 %igen Umsatz beendet.

Um ein sehr reines Endprodukt zu erhalten, ist es vorteilhaft, dass der nach Schritt d) erhaltene Rückstand mit Hilfe von Alkalimetallalkoholat eines aliphatische C₁- bis C₄-Alkohols, vorzugsweise mit Natriummethylat, Natriumethylat,- Kaliummethylat oder Kaliumethylat, oder von Alkalimetallhydroxid neutralisiert wird, Alkohol oder Alkohol und/oder Wasser durch Anlegen von Vakuum abgezogen wird und das im Rückstand verbleibende vicinale Alkandiol oder Alkantriol destillativ isoliert und ggf. gereinigt. Falls notwendig erfolgt die weitere Aufreinigung der gebildeten Diole bzw. Triole vorzugsweise durch Destillation. Um die thermische Belastung der Zielprodukte möglichst niedrig zu halten wird eine kontinuierliche Destillation beispielsweise in einem Falllfilm-, Dünnschicht- oder Kurzwegverdampfer bevorzugt. Dabei werden hervorragende Qualitäten erhalten, die zudem nahezu bis völlig geruchlos sind und damit direkt für Kosmetikanwendungen geeignet sind.

Erfindungsgemäß vorteilhaft ist es, wenn das bei der Destillation gemäß Schritt b) anfallende Ameisensäure/Wasser-Gemisch auf eine gewünschte Konzentration von 50 bis 100 % Ameisensäure aufkonzentriert wird und in der Umsetzung gemäß Schritt a1) oder a2) wiederverwendet wird.

Ebenfalls vorteilhaft ist es, wenn der Alkohol aus dem bei der Destillation gemäß Schritt d) anfallenden Ameisensäureester/Alkoholgemisches abgetrennt wird und in Schritt c) wiederverwendet wird.

Das erfindungsgemäße Verfahren kann sowohl kontinuierlich aber auch diskontinuierlich durchgeführt werden. Dabei ist es insbesondere vorteilhaft, wenn die Umesterung gemäß Schritt c) und das Abdestillieren gemäß Schritt d) kontinuierlich durchgeführt werden.

Mit Hilfe des erfindungsgemäßen Verfahrens gelingt es, hochreine und geruchsfreie Alkandiole oder Alkantriole zu erhalten. Dabei werden Reinheiten von > 99,0 % und sogar von > 99,5 nach Destillation der Diole oder Triole erzielt.

Ein großer Vorteil des erfindungsgemäßen Verfahrens ist, dass es sehr einfach in seiner Durchführung ist und mit den in der Technik üblichen Vorrichtungen durchführbar ist und darüber hinaus nur vergleichsweise geringe Mengen an Hilfsstoffen wie Alkohol oder Säure eingesetzt werden müssen, die zudem noch leicht abgetrennt und ggf. regeneriert und recycliert werden können.

Durch das erfindungsgemäße Verfahren wird eine destillative Reinigung von Diolen bzw. Triolen mit einem wirtschaftlich vertretbaren Aufwand zu hohen Reinheitsgraden und in nahezu bis völlig geruchloser Qualität und damit für Kosmetikanwendungen überhaupt erst ermöglicht.

Die nach dem erfindungsgemäßen Verfahren herstellbaren vicinalen Alkandiole und Alkantriole sind daher hervorragend geeignet für die Verwendung als Bestandteil einer kosmetischen Formulierung, beispielsweise als Feuchthaltemittel etwa in Cremes. Gegenstand der Erfindung ist daher auch die Verwendung von vicinalen Alkandiolen und Alkantriolen hergestellt nach dem oben beschriebenen Verfahren in kosmetischen Formulierungen, insbesondere als Feuchthaltemittel.

Die im Folgenden aufgeführte Beispiele sollen die Erfindung weiter verdeutlichen aber nicht einschränken.

### Beispiel 1: 1,2,6-Hexantriol

In einem 2 l ummantelten Glasreaktor mit Glasrührer, Tropftrichter zur H₂O₂-Dosierung, 0,5 m langer Füllkörperkolonne, Dampfteiler, Kühler, Destillatvorlage und angeschlossener Abgasmessung wurden 450 g (4,22 mol) 5-Hexen-1-ol (94 % ige technische Ware) vorgelegt, bei Raumtemperatur 1059 g (23,0 mol) HCOOH zugegeben, auf 55 °C eingestellt und 1 Stunde bei 55 °C gehalten. Anschließend wurden 373 g (5,0 mol) Wasserstoffperoxid (50 %ige wässrige Lösung) innerhalb von 6 Stunden zudosiert. Der Reaktionsverlauf war exotherm. Die Reaktortemperatur wurde bei 55 bis max. 58 °C gehalten. Nach einer Nachreaktionszeit von ca. 3 Stunden bei 55 °C war die Umsetzung beendet, die Umsatzkontrolle erfolgte gaschromatographisch.

Während der Peroxiddosierung und Nachreaktion entstanden < 0,5 l Abgas (überwiegend O₂). Die Peroxidkonzentration der Reaktionslösung lag zu diesem Zeitpunkt bei ca. 1,5 %. Die höchste Peroxidkonzentration wurde am Ende der Peroxiddosierung mit 2,5 bis 3,5 % gemessen.

Zur Peroxidzerstörung wurde der Reaktorinhalt auf 80 °C aufgeheizt und 1 Stunde bei dieser Temperatur gehalten. Es entstanden ca. 10 l Abgas (überwiegend O₂) und der Peroxidgehalt sank dabei auf < 0,1 %.

Durch Anlegen von Wasserstrahlvakuum wurde nun H₂O/HCOOH aus dem Reaktionsgemisch abgezogen und dabei die Peroxidkonzentration noch weiter abgesenkt (< 0,05 %). Bei dem verbleibenden Sumpf (1019 g) handelte es sich um ein Alkantriol-mono, di- und triformiatgemisch. Zur Umesterung wurde der Sumpf mit 3,8 g (0,012 mol) Marlon-AS3-Säure und 764 g Methanol versetzt und auf 60 - 70 °C aufgeheizt. Das dabei entstandene Methylformiat wurde mit überschüssigem Methanol über Kopf unter Normaldruck abgezogen. Nach einer Verweilzeit von 4 Stunden war nahezu vollständiger Formiat-Umsatz erreicht (Verseifungszahl: > 99,5 %iger Esterumsatz). Anschließend wurden zur Neutralisation des Katalysators 2,8 g NM30 (30 %ige methanolische Natriummethylat-Lösung) zugegeben und restliches Methanol durch Anheben der Reaktortemperatur auf 82 °C und Absenken des Druckes auf 500 hPa abgezogen.
Die anschließende destillative Aufarbeitung erfolgte unter schonenden Destillationsbedingungen bei einer maximalen Sumpftemperatur von 220 °C. Es wurde eine nahezu geruchlose, farblos-klare Hauptlauffraktion von 476 g mit einem 1,2,6-Hexantriol-Gehalt von > 99 % erhalten. Die Ausbeute an Reinprodukt betrug 84 % der Theorie bezogen auf eingesetztes 5-Hexen-1-ol. Die Gesamtausbeute inklusive der in Zwischenfraktionen enthaltenen 1,2,6-Hexantriols betrug 92 % der Theorie.
Die Zwischenfraktionen wurden nach mehreren Wiederholungen gesammelt und erneut destilliert, wobei wiederum reines 1,2,6-Hexantriol erhalten wurde. Somit gelang es über alle Ansätze gerechnet die Ausbeute an isoliertem Reinprodukt bis in die Nähe der Gesamtausbeute zu erhöhen.

### Beispiel 2: 1,2,6-Hexantriol

Die Herstellung des Hexantriol-Estergemisches erfolgte entsprechend dem Beispiel 1. Zur nachfolgenden Umesterung wurden aber anstelle von Methanol, 1653 g n-Butanol eingesetzt und die Reaktion bei 125 - 130 °C in 5 Stunden durchgeführt. Die anschließende Aufarbeitung erfolgte entsprechend dem Beispiel 1 und es wurde eine Hauptlauffraktion mit vergleichbarer Produktqualität und Ausbeute an 1,2,6-Hexantriol erzielt.

### Beispiel 3: 1,2,6-Hexantriol (nicht erfindungsgemäß)

Die Herstellung des Hexantriol-Estergemisches erfolgte entsprechend dem Beispiel 1. Zur Umesterung mit Methanol wurde anstelle von Marlon-AS3-Säure, 0,004 mol Schwefelsäure als Katalysator eingesetzt. Nach der anschließenden Aufarbeitung wurde eine > 99 %ige Hauptlauffraktion an 1,2,6-Hexantriol mit einem stechenden Geruch erhalten.

### Beispiel 4: 1,2-Hexandiol

242 g (2,87 mol) 1-Hexen wurden mit 330 g Ameisensäure (7,17 mol) und 232 g (3,41 mol) einer 50 %igen wässrigen H₂O₂-Lösung analog zu Beispiel 1 umgesetzt. Dabei wurde jedoch direkt nach der Ameisensäurezugabe und Einstellung der Reaktortemperatur auf 55 °C mit der Wasserstoffperoxid-Dosierung begonnen und diese bei 55 - 60°C innerhalb von 6 Stunden durchgeführt. Nach der H₂O₂-Dosierung und Nachreaktion (3 Stunden bei 55 °C) wurde direkt die destillative H₂O/HCOOH-Abtrennung bei 70 - 138 °C im Wasserstrahlvakuum durchgeführt.
Die Umesterung wurde in Gegenwart von 0,001 mol Marlon-AS3-Säure und mit 400 g Methanol bei 55 - 85 °C in 3 Stunden durchgeführt und nach der destillativen Aufarbeitung als Hauptlauffraktion > 99,5 % iges, nahezu geruchloses 1,2-Hexandiol erhalten.
Die 1,2-Hexandiol-Ausbeute bezogen auf eingesetztes 1-Hexen betrug: 92 % der Theorie (gesamt) bzw. 80 % der Theorie (Reinprodukt).

### Bedingungen:

| | |
|---|---|
| Umsetzung mit H₂O₂ (Dosierung) : | 55 - 60°C / 6 Stunden |
| Nachreaktion: | 65 °C / 3 Stunden |
| H₂O/ HCOOH-Abtrennung unter Vakuum: | 70 - 138 °C |
| Umesterung: | 55 - 85 °C / 3 Stunden |

### Beispiel 5: 1,2-Octandiol

314 g (2,80 mol) 1-Octen wurden mit 330 g (7,17 mol) Ameisensäure und 232 g (3,41 mol) einer 50 %igen wässrigen H₂O₂-Lösung entsprechend dem Beispiel 4 umgesetzt. Zur Marlon-AS3-Säure-katalysierten Umesterung werden 575 g Ethanol eingesetzt und nach der destillativen Aufarbeitung als Hauptlauffraktion > 99,5 % iges, nahezu geruchloses 1,2-Octandiol erhalten.
Die 1,2-Octandiol-Ausbeute bezogen auf eingesetztes 1-Octen betrug: 85 % der Theorie (gesamt) bzw. 75 % der Theorie (Reinprodukt).

### Bedingungen:

| | |
|---|---|
| Umsetzung mit H₂O₂ (Dosierung) : | 55 °C / 6 Stunden |
| Nachreaktion: | 65 °C / 3 Stunden |
| H₂O/ HCOOH-Abtrennung unter Vakuum: | 65 - 110 °C |
| Umesterung: | 75 - 85 °C / 3 Stunden |

### Beispiel 6: 1,2-Dodecandiol

336 g (2,00 mol) 1-Dodecen wurden mit 236 g Ameisensäure und 185 g einer 50 %igen wässrigen H₂O₂-Lösung entsprechend dem Beispiel 4 umgesetzt. Zur Marlon-AS3-Säure-katalysierten Umesterung wurden 412 g Ethanol eingesetzt und nach der destillativen Aufarbeitung als Hauptlauffraktion 99,5 % iges, nahezu geruchloses 1,2-Dodecandiol erhalten.

Die 1,2-Dodecandiol-Ausbeute bezogen auf eingesetztes 1-Dodecen betrug: 82 % der Theorie (gesamt) bzw. 74 % der Theorie (Reinprodukt).

### Bedingungen:

| | |
|---|---|
| Umsetzung mit H₂O₂ (Dosierung) | 55 °C / 8 Stunden |
| Nachreaktion: | 65 °C / 3 Stunden |
| H₂O/ HCOOH-Abtrennung unter Vakuum: | 65 - 115 °C |
| Umesterung: | 75 - 85 °C / 3 Stunden |

### Beispiel 7: 1,2,4-Butantriol

72,1 g (1,00 mol) 3-Buten-1-ol wurden mit 120 g (2,61 mol) Ameisensäure und 82 g (1,21 mol) einer 50 %iger wässriger H₂O₂-Lösung entsprechend dem Beispiel 1 umgesetzt. Zur Marlon-AS3-Säure-katalysierten Umesterung wurden 100 g Methanol eingesetzt und nach der destillativen Aufarbeitung als Hauptlauffraktion > 96,3 % iges, nahezu geruchloses 1,2,4-Butantriol erhalten.
Die 1,2,4-Butantriol-Ausbeute bezogen auf eingesetztes 3-Buten-1-ol betrug: 72 % der Theorie (gesamt) bzw. 70 % der Theorie (Reinprodukt).

### Bedingungen:

| | |
|---|---|
| Veresterung 3-Buten-1-ol + HCOOH: | 55 °C / 1 Stunde |
| Umsetzung mit H₂O₂ (Dosierung) : | 55 - 60 °C /4 Stunden |
| Nachreaktion: | 55 °C /2 Stunden |
| Peroxidzerstörung: | 80 °C /2 Stunden |
| H₂O/ HCOOH-Abtrennung unter Vakuum: | 65 - 115 °C |
| Umesterung: | 55 - 85 °C /3 Stunden |

### Beispiel 8: 1,2-Dodecandiol (Vergleich gemäß DE 197 43 015, Beispiel 6, nicht erfindungsgemäß)

168,3 g (1,0 mol) 1-Dodecen und 73,6 g (1,6 mol) Ameisensäure wurden vorgelegt. Man erwärmte diese Mischung auf 100 °C und tropfte anschließend 115,7 g (1,7 mol) 50 %ige Wasserstoffperoxidlösung über einen Zeitraum von 2 Stunden zu. Anschließend rührte man bei 100 °C noch 45 min nach. Man versetzte mit 64,1 g (2 mol) Methanol und 0,1 g konzentrierter Schwefelsäure und erhitzte 30 min. unter Rückfluss und destillierte anschließend bei Normaldruck den Ameisensäuremethylester, Methanol und Wasser ab.
Nach der anschließenden destillativen Aufarbeitung wurde eine > 99 %ige Hauptlauffraktion isoliert. Das frisch destillierte Zielprodukt hatte einen unangenehmen, stechenden Geruch.
Die 1,2-Dodecandiol-Ausbeute bezogen auf eingesetztes 1-Dodecen betrug: 81 % der Theorie (gesamt) bzw. 73 % der Theorie (Reinprodukt).

### Beispiel 9: 1,2,6-Hexantriol

Die Herstellung des Hexantriol-Estergemisches erfolgte entsprechend dem Beispiel 1. Zur nachfolgenden Umesterung mit Ethanol (1098 g) wurde anstelle von Marlon-AS3-Säure als Katalysator 2 g (0,012 mol) Hexansulfonsäure eingesetzt. Die anschließende Aufarbeitung erfolgte entsprechend dem Beispiel 1 und es wurde eine Hauptlauffraktion mit vergleichbarer Produktqualität und Ausbeute an 1,2,6-Hexantriol erzielt.

### Beispiel 10: 1,2,6-Hexantriol

Die Herstellung des Hexantriol-Estergemisches erfolgte entsprechend dem Beispiel 9. Zur nachfolgenden Umesterung mit Ethanol wurde anstelle von Hexansulfonsäure als Katalysator 3,2 g (0,012 mol) Dodecansulfonsäure eingesetzt. Die anschließende Aufarbeitung erfolgte entsprechend dem Beispiel 9 und es wurde eine Hauptlauffraktion mit vergleichbarer Produktqualität und Ausbeute an 1,2,6-Hexantriol erzielt.

## Patentansprüche

1. Verfahren zur Herstellung von vicinalen Alkandiolen oder Alkantriolen umfassend die Schritte
a1) Umsetzung entsprechender Monoolefine mit 4 bis 20 Kohlenstoffatomen mit Wasserstoffperoxid und Ameisensäure in Gegenwart von Wasser oder
a2) Umsetzung entsprechender Monoolefinalkohole mit 4 bis 20 Kohlenstoffatomen in einer ersten Stufe mit Ameisensäure und des in der ersten Stufe gebildeten Reaktionsproduktes in einer zweiten Stufe mit Wasserstoffperoxid in Gegenwart von Wasser,
b) Abdestillieren von Wasser und Ameisensäure aus der in Schritt a1) oder a2) erhaltenen Mischung,
c) Umsetzung der in Schritt b) erhaltenen Mischung mit mindestens einem aliphatischen C₁-C₄-Alkohol in Gegenwart von mindestens einer Arylsulfonsäure der Formel RₙAr-SO₃H, bei der R gleich oder verschieden sein können und einen linearen oder verzweigten C₁- bis C₁₉-Alkylrest, Fluor oder Chlor bedeuten, Ar ein Phenyl-, Naphthyl-, Anthryl- oder Phenanthrylrest ist und n = 0, 1, 2 oder 3 sein kann, oder von mindestens einer C₆- bis C₁₆-Alkylsulfonsäure, und
d) Abdestillieren von in Schritt c) gebildetem Ameisensäureester zusammen mit ggf. noch vorhandenem Alkohol aus der Reaktionsmischung von Schritt c),
wobei die Schritte c) und d) nacheinander oder gleichzeitig durchgeführt werden.

2. Verfahren nach Anspruch 1,
**dadurch gekennzeichnet,**
**dass** in Schritt a1) 2,4,4-Trimethyl-1-penten, 2,4,4-Trimethyl-2-penten oder Gemische aus beiden Isomeren, 1-Penten, 1-Hexen, 1-Octen oder 1-Dodecen eingesetzt werden.

3. Verfahren nach Anspruch 1,
**dadurch gekennzeichnet,**
**dass** in Schritt a2) 3-Buten-1-ol, 5-Hexen-1-ol oder 9-Decen-1-ol eingesetzt werden.

4. Verfahren nach einem der vorhergehenden Ansprüche,
**dadurch gekennzeichnet,**
**dass** in Schritt a2) mindestens 2 Molequivalente Ameisensäure eingesetzt werden.

5. Verfahren nach einem der vorhergehenden Ansprüche,
**dadurch gekennzeichnet,**
**dass** Schritt a1) oder die zweite Stufe von Schritt a2) bei Temperaturen von 40 bis 120°C durchgeführt werden.

6. Verfahren nach einem der vorhergehenden Ansprüche,
**dadurch gekennzeichnet,**
**dass** das in Schritt a1) oder in Schritt a2) eingesetzte Wasserstoffperoxid in Form einer 30 - 70 %igen wässrigen Lösung eingesetzt wird.

7. Verfahren nach einem der vorhergehenden Ansprüche,
**dadurch gekennzeichnet,**
**dass** in Schritt b) das Abdestillieren von Wasser und Ameisensäure bei einem Druck von 100 mbar bis 1 bar und einer Sumpftemperatur von 20 bis 150°C durchgeführt wird.

8. Verfahren nach Anspruch 7,
**dadurch gekennzeichnet,**
**dass** das Abdestillieren von Wasser und Ameisensäure bei einem Druck von 200 mbar bis 800 mbar und einer Sumpftemperatur von 60 bis 120°C durchgeführt wird.

9. Verfahren nach einem der vorhergehenden Ansprüche,
**dadurch gekennzeichnet,**
**dass** die in Schritt b) erhaltene Mischung vor Schritt c) thermisch behandelt wird zur Zerstörung noch vorhandenen Wasserstoffperoxids oder organischer Hydroperoxide.

10. Verfahren nach einem der vorhergehenden Ansprüche,
**dadurch gekennzeichnet,**
**dass** in Schritt c) als aliphatischer C₁-C₄-Alkohol Methanol, Ethanol, n-Propanol, iso-Propanol, n-Butanol, sek.-Butanol oder tert.-Butanol eingesetzt wird.

11. Verfahren nach Anspruch 10,
**dadurch gekennzeichnet,**
**dass** Ethanol, n-Propanol und/oder iso-Propanol eingesetzt werden.

12. Verfahren nach einem vorhergehenden Ansprüche,
**dadurch gekennzeichnet,**
**dass** in Schritt c) als Arylsulfonsäure Benzolsulfonsäure, p-Toluolsulfonsäure und/oder Marlon-AS3-Säure bzw. als Alkylsulfonsäure Hexansulfonsäure, und/oder Dodecansulfonsäure eingesetzt werden.

13. Verfahren nach Anspruch 12,
**dadurch gekennzeichnet,**
**dass** Marlon-AS3-Säure eingesetzt wird.

14. Verfahren nach einem der vorhergehenden Ansprüche,
**dadurch gekennzeichnet,**
**dass** die Menge an Katalysator in Schritt c) von 0,005 bis 1 Mol.-% bezogen auf die Menge an eingesetztem Monoolefin bzw. Monoolefinalkohol verwendet wird.

15. Verfahren nach einem der vorhergehenden Ansprüche,
**dadurch gekennzeichnet,**
**dass** die Umsetzung mit C₁-C₄-Alkohol in Schritt c) und die Abtrennung des Ameisensäureesters bei einer Sumpftemperatur von 40 bis 160 °C erfolgt.

16. Verfahren nach Anspruch 15,
**dadurch gekennzeichnet,**
**dass** die Umsetzung mit C₁-C₄-Alkohol in Schritt c) und die Abtrennung des Ameisensäureesters bei einer Sumpftemperatur von 50 bis 110 °C erfolgt.

17. Verfahren nach einem der vorhergehenden Ansprüche,
**dadurch gekennzeichnet,**
**dass** der nach Schritt d) erhaltene Rückstand mit Hilfe von Alkalimetallalkoholat eines aliphatische C₁- bis C₄-Alkohols oder von Alkalimetallhydroxid neutralisiert wird, Alkohol oder Alkohol und/oder Wasser durch Anlegen von Vakuum abgezogen wird und das im Rückstand verbleibende vicinale Alkandiol oder Alkantriol destillativ isoliert wird.

18. Verfahren nach Anspruch 17,
**dadurch gekennzeichnet,**
**dass** mit Natriummethylat, Natriumethylat,- Kaliummethylat oder Kaliumethylat neutralisiert wird.

19. Verfahren nach einem der vorhergehenden Ansprüche,
**dadurch gekennzeichnet,**
**dass** das bei der Destillation gemäß Schritt b) anfallende Ameisensäure/Wasser-Gemisch auf eine gewünschte Konzentration von 50 bis 100 % Ameisensäure aufkonzentriert wird und in der Umsetzung gemäß Schritt a1) oder a2) wiederverwendet wird.

20. Verfahren nach einem der vorhergehenden Ansprüche,
**dadurch gekennzeichnet,**
**dass** der Alkohol aus dem bei der Destillation gemäß Schritt d) anfallenden Ameisensäureester/Alkoholgemisches abgetrennt wird und in Schritt c) wiederverwendet wird.

21. Verfahren nach einem der vorhergehenden Ansprüche,
**dadurch gekennzeichnet,**
**dass** die Umesterung gemäß Schritt c) und das Abdestillieren gemäß Schritt d) kontinuierlich durchgeführt werden.

## Claims

1. Process for preparing vicinal alkanediols or alkanetriols comprising the steps of
a1) reaction of appropriate monoolefins having 4 to 20 carbon atoms with hydrogen peroxide and formic acid in the presence of water or
a2) reaction of appropriate monoolefin alcohols having 4 to 20 carbon atoms in a first stage with formic acid and of the reaction product formed in the first stage with hydrogen peroxide in the presence of water in a second stage,
b) removal of water and formic acid by distillation from the mixture obtained in step a1) or a2),
c) reaction of the mixture obtained in step b) with at least one aliphatic C₁-C₄-alcohol in the presence of at least one arylsulfonic acid of the formula RₙAr-SO₃H, in which R may be identical or different and is a linear or branched C₁- to C₁₉-alkyl radical, fluorine or chlorine, Ar is a phenyl, naphthyl, anthryl or phenanthryl radical, and n can be 0, 1, 2 or 3, or of at least one C₆- to C₁₆-alkylsulfonic acid and
d) removal by distillation of the formic ester formed in step c) together with alcohol which is still present where appropriate from the reaction mixture from step c),
in which steps c) and d) are carried out successively or simultaneously.

2. Process according to Claim 1, **characterized in that** 2,4,4-trimethyl-1-pentene, 2,4,4-trimethyl-2-pentene or mixtures of the two isomers, 1-pentene, 1-hexene, 1-octene or 1-dodecene are employed in step a1).

3. Process according to Claim 1, **characterized in that** 3-buten-1-ol, 5-hexen-1-ol or 9-decen-1-ol are employed in step a2).

4. Process according to any of the preceding claims, **characterized in that** at least 2 mole equivalents of formic acid are employed in step a2).

5. Process according to any of the preceding claims, **characterized in that** step a1) or the second stage of step a2) are carried out at temperatures of from 40 to 120°C.

6. Process according to any of the preceding claims, **characterized in that** the hydrogen peroxide employed in step a1) or in step a2) is employed in the form of a 30-70% strength aqueous solution.

7. Process according to any of the preceding claims, **characterized in that** the removal of water and formic acid by distillation in step b) is carried out under a pressure of from 100 mbar to 1 bar and at a bottom temperature of from 20 to 150°C.

8. Process according to Claim 7, **characterized in that** the removal of water and formic acid by distillation is carried out under a pressure of from 200 mbar to 800 mbar and at a bottom temperature of from 60 to 120°C.

9. Process according to any of the preceding claims, **characterized in that** the mixture obtained in step b) is thermally treated before step c) to decompose hydrogen peroxide which is still present or organic hydroperoxides.

10. Process according to any of the preceding claims, **characterized in that** methanol, ethanol, n-propanol, isopropanol, n-butanol, sec-butanol or tert-butanol is employed as aliphatic C₁-C₄-alcohol in step c).

11. Process according to Claim 10, **characterized in that** ethanol, n-propanol and/or isopropanol are employed.

12. Process according to any of the preceding claims, **characterized in that** benzenesulfonic acid, p-toluenesulfonic acid and/or Marlon AS3 acid are employed as arylsulfonic acid, or hexanesulfonic acid and/or dodecanesulfonic acid are employed as alkylsulfonic acid, in step c).

13. Process according to Claim 12, **characterized in that** Marlon AS3 acid is employed.

14. Process according to any of the preceding claims, **characterized in that** the amount of catalyst used in step c) is from 0.005 to 1 mol%, based on the amount of monoolefin or monoolefin alcohol employed.

15. Process according to any of the preceding claims, **characterized in that** the reaction with C₁-C₄-alcohol in step c) and the removal of the formic ester take place at a bottom temperature of from 40 to 160°C.

16. Process according to Claim 15, **characterized in that** the reaction with C₁-C₄-alcohol in step c) and the removal of the formic ester take place at a bottom temperature of from 50 to 110°C.

17. Process according to any of the preceding claims, **characterized in that** the residue obtained after step d) is neutralized with the aid of alkali metal alcoholate of an aliphatic C₁- to C₄-alcohol or with the aid of alkali metal hydroxide, alcohol or alcohol and/or water is stripped off by applying vacuum, and the vicinal alkanediol or alkanetriol remaining in the residue is isolated by distillation.

18. Process according to Claim 17, **characterized in that** sodium methoxide, sodium ethoxide, potassium methoxide or potassium ethoxide is used for neutralization.

19. Process according to any of the preceding claims, **characterized in that** the formic acid/water mixture resulting from the distillation in step b) is concentrated to a desired concentration of from 50 to 100% formic acid and is reused in the reaction in step a1) or a2).

20. Process according to any of the preceding claims, **characterized in that** the alcohol is removed from the formic ester/alcohol mixture resulting from the distillation in step d) and is reused in step c).

21. Process according to any of the preceding claims, **characterized in that** the transesterification in step c) and the removal by distillation in step d) are carried out continuously.

## Revendications

1. Procédé de préparation d'alcanediols ou alcanetriols vicinaux, comprenant les étapes consistant à :
a1) faire réagir des monooléfines correspondantes ayant de 4 à 20 atomes de carbone avec du peroxyde d'hydrogène et de l'acide formique en présence d'eau, ou
a2) faire réagir des alcools monooléfiniques correspondants ayant de 4 à 20 atomes de carbone dans un premier palier avec de l'acide formique et faire réagir le produit réactionnel formé dans le premier palier avec du peroxyde d'hydrogène en présence d'eau dans un deuxième palier,
b) séparer par distillation l'eau et l'acide formique du mélange obtenu à l'étape a1) ou a2),
c) faire réagir le mélange obtenu à l'étape b) avec au moins un alcool aliphatique en C₁ à C₄ en présence d'au moins un acide arylsulfonique de formule RₙAr-SO₃H, où R peuvent être identique ou différent et représentent un résidu alkyle en C₁ à C₁₉ linéaire ou ramifié, du fluor ou du chlore, Ar est un résidu phényle, naphtyle, anthryle ou phénanthryle, et n peut valoir 0, 1, 2 ou 3, ou en présence d'au moins un acide alkylsulfonique en C₆ à C₁₆, et
d) séparer par distillation l'ester d'acide formique formé à l'étape c) ensemble avec l'alcool le cas échéant encore présent du mélange réactionnel de l'étape c),
les étapes c) et d) étant effectuées à la suite l'une de l'autre ou simultanément.

2. Procédé selon la revendication 1, **caractérisé en ce que**, à l'étape a1), on utilise du 2,4,4-triméthyl-1-pentène, du 2,4,4-triméthyl-2-pentène ou des mélanges de ces deux isomères, du 1-pentène, du 1-hexène, du 1-octène ou du 1-dodécène.

3. Procédé selon la revendication 1, **caractérisé en ce que**, à l'étape a2), on utilise du 3-butén-1-ol, du 5-hexén-1-ol ou du 9-décén-1-ol.

4. Procédé selon l'une des revendications précédentes, **caractérisé en ce que**, à l'étape a2), on utilise au moins 2 équivalents molaires d'acide formique.

5. Procédé selon l'une des revendications précédentes, **caractérisé en ce que** l'étape a1) ou le deuxième palier de l'étape a2) se fait à des températures allant de 40 à 120°C.

6. Procédé selon l'une des revendications précédentes, **caractérisé en ce que** le peroxyde d'hydrogène utilisé à l'étape a1) ou à l'étape a2) est mis en oeuvre sous la forme d'une solution aqueuse à 30% à 70%.

7. Procédé selon l'une des revendications précédentes, **caractérisé en ce que**, à l'étape b), la séparation de l'eau et de l'acide formique par distillation se fait sous une pression allant de 100 mbar à 1 bar et à une température de bas de colonne allant de 20 à 150°C.

8. Procédé selon la revendication 7, **caractérisé en ce que** la séparation de l'eau et de l'acide formique par distillation se fait sous une pression allant de 200 mbar à 800 mbar et à une température de bas de colonne allant de 60 à 120°C.

9. Procédé selon l'une des revendications précédentes, **caractérisé en ce que** le mélange obtenu à l'étape b) est soumis avant l'étape c) à un traitement thermique afin de détruire le peroxyde d'hydrogène ou les hydroperoxydes organiques encore présents.

10. Procédé selon l'une des revendications précédentes, **caractérisé en ce que**, à l'étape c), on utilise comme alcool aliphatique en C₁ à C₄ le méthanol, l'éthanol, le n-propanol, l'isopropanol, le n-butanol, le sec-butanol ou le tert-butanol.

11. Procédé selon la revendication 10, **caractérisé en ce que** l'on utilise de l'éthanol, du n-propanol et/ou de l'isopropanol.

12. Procédé selon l'une des revendications précédentes, **caractérisé en ce que**, à l'étape c), on utilise comme acide arylsulfonique l'acide benzènesulfonique, l'acide p-toluènesulfonique et/ou l'acide de Marlon AS3 ou bien comme acide alkylsulfonique l'acide hexanesulfonique, et/ou l'acide dodécanesulfonique.

13. Procédé selon la revendication 12, **caractérisé en ce que** l'on utilise de l'acide de Marlon AS3.

14. Procédé selon l'une des revendications précédentes, **caractérisé en ce que**, à l'étape c), la quantité de catalyseur va de 0,005 à 1 % molaires, rapportée à la quantité de monooléfine ou d'alcool monooléfinique utilisée.

15. Procédé selon l'une des revendications précédentes, **caractérisé en ce que** la réaction avec l'alcool en C₁ à C₄ à l'étape c) et la séparation de l'ester d'acide formique se font à une température de bas de colonne allant de 40 à 160°C.

16. Procédé selon la revendication 15, **caractérisé en ce que** la réaction avec l'alcool en C₁ à C₄ à l'étape c) et la séparation de l'ester d'acide formigue se font à une température de bas de colonne allant de 50 à 110°C.

17. Procédé selon l'une des revendications précédentes, **caractérisé en ce que** le résidu obtenu après l'étape d) est neutralisé au moyen d'un alcoolate de métal alcalin d'un alcool aliphatique en C₁ à C₄ ou d'un hydroxyde de métal alcalin, l'alcool ou l'alcool et/ou l'eau sont enlevés en appliquant un vide et l'alcanediol ou alcanetriol vicinal restant dans le résidu est isolé par distillation.

18. Procédé selon la revendication 17, **caractérisé en ce que** la neutralisation se fait au moyen de méthylate de sodium, d'éthylate de sodium, de méthylate de potassium ou d'éthylate de potassium.

19. Procédé selon l'une des revendications précédentes, **caractérisé en ce que** le mélange acide formique/eau qui résulte de la distillation suivant l'étape b) est concentré à une concentration en acide formique voulue de 50 à 100% et réutilisé dans la réaction de l'étape a1) ou a2).

20. Procédé selon l'une des revendications précédentes, **caractérisé en ce que** l'alcool est séparé du mélange ester d'acide formique/alcool résultant de la distillation suivant l'étape d) et réutilisé à l'étape c).

21. Procédé selon l'une des revendications précédentes, **caractérisé en ce que** la transestérification suivant l'étape c) et la séparation par distillation suivant l'étape d) se font en continu.
